# EUROPÄISCHE PATENTSCHRIFT

(11) **EP 1 687 031 B1**
(45) Veröffentlichungstag und Bekanntmachung des Hinweises auf die Patenterteilung: **17.02.2010**
(21) Anmeldenummer: 04820387.1
(22) Anmeldetag: 26.10.2004
(51) Int. Cl.: A61K 47/18, A61K 47/32, A61K 47/30, A61K 47/02, A61K 47/06, A61K 39/395, A61K 9/08

(54) **PHARMAZEUTISCHE ZUBEREITUNG ENTHALTEND EINEN ANTIKÖRPER GEGEN DEN REZEPTOR FÜR EINEN EPIDERMALEN WACHSTUMSFAKTOR (EGF-REZEPTOR)**
PHARMACEUTICAL PREPARATION CONTAINING AN ANTIBODY FOR THE EPIDERMAL GROWTH FACTOR (EGF) RECEPTOR
PRÉPARATION PHARMACEUTIQUE CONTENANT UN ANTICORPS CONTRE LE RÉCEPTEUR DU FACTEUR DE CROISSANCE ÉPIDERMIQUE (EGF)

(30) Priorität: 26.11.2003 DE 10355251
(43) Veröffentlichungstag der Anmeldung: 09.08.2006
(73) Patentinhaber: Merck Patent GmbH, 64293 Darmstadt (DE)
(72) Erfinder: MAHLER, Hanns-Christian, 65205 Wiesbaden (DE); MÜLLER, Robert, 4056 Basel (CH)
(86) Internationale Anmeldenummer: PCT/EP2004/012044
(87) Internationale Veröffentlichungsnummer: WO 2005/058365

(56) Entgegenhaltungen:
- EP-A- 0 852 951
- WO-A-02/072636
- WO-A-2004/060343
- DE-A1- 10 163 459

## Beschreibung

Die vorliegende Erfindung betrifft eine stabile pharmazeutische Zubereitung enthaltend einen Antikörper, der gegen den Rezeptor des epidermalen Wachstumsfaktors (EGFR) gerichtet ist, deren Herstellung und deren Verwendung.

In verschiedenen in vitro und in vivo Studien konnte gezeigt werden, dass die Blockade des EGFR durch Antikörper auf unterschiedlichen Ebenen gegen Tumore wirkt, beispielsweise durch Hemmung der Krebszellproliferation, Verringerung der tumorvermittelten Angiogenese, Induktion der Krebszellapoptose und Verstärkung der toxischen Wirkungen der Strahlentherapie und der herkömmlichen Chemotherapie.

MAB c225 (INN: Cetuximab) ist ein klinisch erprobter Antikörper, der an den EGF-Rezeptor bindet. Cetuximab ist ein chimärer Antikörper, dessen variable Regionen murinen und dessen konstante Regionen humanen Ursprungs sind. Cetuximab wurde erstmals von Naramura et al., Cancer Immunol Immunotherapy 1993, 37: 343-349 sowie in WO 96/40210 A1 beschrieben.

MAB 425 ist ein ursprünglich muriner Antikörper, der gegen den auf TumorZellen, insbesondere auf A431 Carcinoma-Zellen, überexprimierten EGFR gerichtet ist. Seine humanisierten und chimären Formen sind z.B. aus EP 0 531 472 A1; Kettleborough et al., Protein Engineering 1991, 4: 773-783; Bier et al., Cancer Chemother Pharmacol 2001, 47: 519-524; Bier et al., Cancer Immunol Immunother 1998, 46: 167-173 bekannt. EMD 72000 (h425) ist eine in der klinischen Phase I/II befindliche Form von MAB 425, dessen konstanter Bereich sich aus einer κ und einer humanen γ-1-Kette zusammen setzt.

Humane anti-EGFR-Antikörper können nach der XenoMouse-Technologie bereitgestellt werden, wie beschrieben in WO 91/10741 A1, WO 94/02602 A1, WO 96/33735 A1. Ein gemäß dieser Technologie hergestellter spezieller in der klinischen Erprobung befindlicher Antikörper ist ABX-EGF (Abgenix, Crit Rev Oncol Hematol 2001, 38: 17-23; Cancer Research 1999, 59: 1236-43).

Weitere gegen den EGFR gerichtete Antikörper sind z.B. beschrieben in EP 0 586 002 B1 sowie in J Natl Cancer Inst 1993, 85: 27-33 (MAB 528).

Wie andere Antikörper werden auch die anti-EGFR-Antikörper zur therapeutischen Anwendung parenteral als Lösung appliziert. Ein besonderes Problem von Lösungen mit diesen Antikörpern ist deren Neigung zur Aggregation und zur Bildung von Proteinmultimeren. Im Falle reduzierbarer Multimere kann dies auf nicht beabsichtigte intermolekulare Disulfidbrückenbildung durch eine Interaktion zwischen sich annähernden Molekülteilen zurückgeführt werden. Auch kommen hydrophobe Wechselwirkungen und die damit verbundene Bildung nichtreduzierbarer Multimere in Betracht. Weiterhin kommt es zu Deamidierungsreaktionen, die nachfolgend zu Proteinabbaureaktionen führen. Die beschriebenen Denaturierungsreaktionen treten besonders bei Lagerung bei erhöhter Temperatur auf oder bei Scherbeanspruchung, wie sie beispielweise beim Transport auftritt.

Infolge der genannten Aggregationsneigung kommt es bei Lagerung von Antikörperlösungen zu Produktausfällungen, so dass eine reproduzierbare Entnahme aus dem die Lösung enthaltenden Behältnis in Frage gestellt ist. Hinzu kommt, dass es bei parenteraler Applikation partikelhaltiger Lösung zu Embolien kommen kann. Dies hat zur Folge, dass die Verabreichung der anti-EGFR-Antikörper in der jeweils erforderlichen Dosis mittels Antikörper-Lösungen an den Patienten nicht immer reproduzierbar gewährleistet ist und die Applikation nicht mit der erforderlichen Sicherheit erfolgen kann.

Durch Filtration vor Injektion können die Aggregate zwar zurückgehalten werden. Dieses Verfahren beinhaltet aber einen zusätzlichen Schritt und ist daher aufwendig und für die klinische Praxis wenig geeignet. Auch verbleibt das Problem der Dosisreproduzierbarkeit ungelöst, da jeweils ein unbekannter Anteil an Antikörpern aus der Lösung abgetrennt wird und Partikelbildung nach Filtration weiterhin ein Sicherheitsrisiko darstellt.

Ein gebräuchliches Verfahren zur Stabilisierung von monoklonalen Antikörpern ist die Gefriertrocknung von Lösungen, die Antikörper sowie Hilfsstoffe enthalten. Lyophilisation ist jedoch sehr zeit- und energieaufwendig und damit teuer. Auch muss das Lyophilisat vor Verabreichung erst rekonstituiert werden.

EP 0 073 371 beschreibt intravenös verabreichbare Zubereitungen mit Immunglobulinen, die zur Stabilisierung einen pH-Wert von 3,5 bis 5,0 aufweisen. Derart niedrige pH-Werte führen aber zu unerwünschten Unverträglichkeitsreaktionen an der Injektionsstelle.

US 6,171,586 B1 offenbart die Verwendung von einem Acetatpuffer pH 4,48 bis 5,5, einem Surfactant und einem Polyol in einer wässrigen Formulierung von Antikörpern, wobei NaCl zur Isotonisierung ausgeschlossen ist. Aufgrund der fehlenden Isotonisierung kann es ebenfalls zu Unverträglichkeitsreaktionen an der Injektionsstelle kommen.

Als Beispiele weiterer Formulierungen mit speziellen Antikörpern seien an dieser Stelle EP 0 280 358, EP 0 170 983 und US 5,945,098 genannt.

Hiervon beschreibt EP 0 280 358 den Zusatz von Dextran zu einer Antikörperlösung zur Stabilisierung gegen bestimmte Hormone, wobei eine Stabilität über neun Monate erreicht wurde.

EP 0 170 983 beschreibt die Stabilisierung eines thermolabilen monoklonalen Antikörpers durch Erhitzen zusammen mit hydrolysiertem Ovalbumin, wodurch der Antikörper nach 7 Tagen Lagerung bei 45°C noch stabil war. Der Zusatz von Proteinen anderer Species zu verabreichbaren Formulierungen, die zur parenteralen Verabreichung vorgesehen sind, sind aufgrund der hiermit verbundenen Problematik, insbesondere deren möglichen Antigenität, aber unerwünscht.

US 5,945,098 offenbart die Verwendung von Glycin, Polysorbat 80 und Polyethylenglycol zur Stabilisierung einer wässrigen Formulierung von Immunglobulin G.

DE 10133394 A1 offenbart die Verwendung eines Phosphatpuffers im Bereich von pH 6 bis pH 8 und einem Polyoxyethylensorbitan-Fettsäureester zur Stabilisierung einer wässrigen Lösung des Antikörpers Cetuximab. Obwohl die Bildung sichtbarer Aggregate hierdurch deutlich reduziert ist, wird die chemische Stabilität, insbesondere unter Stressbedingungen, deutlich beeinträchtigt. Ferner zeigt die Formulierung keine Stabilität gegen (extremen) thermischen Stress, z.B. Langzeitlagerung bei 40°C.

DE10163459 legt pharmazeutische Zusammensetzungen offen, die Lyophilisate aus wässriger Lösung oder Wirkstoff-/Puffer/Salz-Lösung darstellen. Sie enthalten folgende Komponenten: a) Antikörper gegen die Rezeptoren des epidermalen Wachstumsfaktors (Cetuximab), b) eine basische, saure oder neutrale Aminosäure in Konzentrationen von 100 mmol (siehe Beispiel 6), c) Tenside (Polyoxyethylen-Sorbitan-Fettsäureester, Polyoxyethylen-Sorbitan-Monooleat (0,01 % in Beispiel 4), Polyoxyethylen-SorbitanMonolaurat, Polyoxyethylen-Polyoxypropylen Polymere), d) möglicherweise Isotonisierungsmittel, wie Natriumchlorid, e) Puffer und f) Zucker oder Aminozucker. Die Lagerstabilität der Lyophilisate wurde erhöht, die Stabilität der hergestellten Lösung zur Applikation blieb unverändert kurz.

WO2004060343 beschreibt die Herstellung von Partikeln, die aus einem Antikörper, wie Cetuximab, einem Puffer, einem Tensid und einer Aminosäure bestehen können.

Es war Aufgabe der Erfindung speziell für die gegen den EGFR gerichtete Antikörper eine zur parenteralen Verabreichung geeignete wässrige Formulierung zu finden, die gut verträglich ist und bei Lagerung bei Raumtemperatur über mindestens 24 Monate stabil ist. Die Lagerstabilität sollte auch durch beim Transport einwirkenden Scherkräften und bei veränderten Klimabedingungen, insbesondere bei erhöhter Temperatur und Luftfeuchtigkeit, erhalten bleiben. Weiterhin sollte die Formulierung einfach aufgebaut sein und keine aus toxikologischer Sicht bedenklichen Hilfsstoffe enthalten.

Überraschenderweise konnte eine diesen Anforderungen entsprechende Formulierung mit einer Lösung gefunden werden, die neben einem gegen den epidermalen Wachstumsfaktor gerichteten Antikörper (anti-EGFR-Antikörper) einen Puffer, eine Aminosäure und ein Tensid enhält. Gegenstand der vorliegenden Erfindung ist daher eine wässrige pharmazeutische Zubereitung, die neben einem anti-EGFR-Antikörper einen Puffer, eine Aminosäure sowie ein Tensid enhält.

Eine wässrige Zubereitung im Sinne der Erfindung liegt vor, wenn zumindest einen Teil des enthaltenen Lösungsmittels aus Wasser besteht. Als weitere Lösungsmittelbestandteile können alle Lösungsmittel enthalten sein, die für die parenterale Anwendung geeignet sind, insbesondere Alkohole wie z. B. Ethanol, Propanol, Propandiol oder Glycerol. Bevorzugt enthält die wässrige Zubereitung als Lösungsmittel Wasser oder Ethanol-Wasser-Gemische, besonders bevorzugt besteht das Lösungsmittel aus Wasser.

Als Antikörper enthalten sein kann jeder anti-EGFR-Antikörper, insbesondere die eingangs genannten murinen, humanisierten und chimären Antikörper sowie die mittels der genannten XenoMouse-Technologie hergestellten und herstellbaren humanen anti-EGFR-Antikörper. Bevorzugt ist der anti-EGFR-Antikörper Cetuximab oder EMD 72000 bzw. eines der diesen entsprechenden murinen, humanisierten oder chimären Antikörperanaloga. Besonders bevorzugt sind wässrige Zubereitungen, die Cetuximab oder EMD 72000 als Antikörper enthalten.

Der anti-EGFR-Antikörper kann in der erfindungsgemäßen Formulierung in einer Konzentration von 0,1 mg/ml bis 50 mg/ml enthalten sein, bevorzugt sind 2 mg/ml bis 10 mg/ml, besonders bevorzugt cirka 5 mg/ml anti-EGFR-Antikörper enthalten.

Als Puffer können grundsätzlich alle physiologisch verträglichen Substanzen eingesetzt werden, die zur Einstellung des gewünschten pH-Wertes geeignet sind, wie z. B. Citrat-Salze, Acetat-Salze, Histidin-Salze Succinat-Salze, Malat-Salze, Phosphat-Salze oder Lactat-Salze und/oder deren jeweilige freien Säuren bzw. Basen sowie Mischungen aus den verschiedenen Salzen und/oder deren Säuren bzw. Basen. Bevorzugt besteht der Puffer aus einem oder mehreren Citrat-Salz/en, Acetat-Salz/en, Histidin-Salz/en, Succinat-Salz/en, Malat-Salz/en, Phosphat-Salz/en oder Lactat-Salz/en und/oder deren jeweilige/n freie/n Säure/n bzw. Base/n oder eine Mischung aus einem oder mehreren der verschiedenen Salze und/oder deren Säure/n bzw. Base/n. Der Ausdruck Mischung umfasst hierbei sowohl Mischungen von verschiedenen Salzen derselben Säure wie z.B. Mischungen verschiedener Citratsalze als auch Mischungen von Salzen verschiedener Säuren wie z.B. Mischungen aus Citrat- und Acetat-Salzen. Bevorzugt besteht der Puffer aus einem oder mehreren Citrat-Salz/en und/oder dessen freie Säure (z.B. Citronensäure, Citronensäure-Monohydrat, tri-Natriumcitrat-Dihydrat, tri-Kaliumcitrat-Monohydrat), Acetat-Salz/en und/oder dessen freie Säure (z.B. Essigsäure, Natriumacetat, Natriumacetat-Trihydrat) oder aus L-Histidin und/oder einem Säureadditionssalz hiervon wie z.B. L-Histidinmonohydrochlorid-Monohydrat. Zweckmäßigerweise enthält die erfindungsgemäße Zubereitung den Puffer in einer Konzentration von 10 bis 100 mmol/l, bevorzugt sind 2 bis 20 mmol/l, besonders bevorzugt sind ca. 10 mmol/l enthalten.

Der pH-Wert der Zubereitung liegt im Bereich von 5,0 bis 6,0, bevorzugt ist ein pH-Wert von 5,2 bis 5,8, besonders bevorzugt ein pH-Wert von cirka 5,5.

Die erfindungsgemäße Zubereitung ist physiologisch gut verträglich, leicht herstellbar, exakt dosierbar und über die Dauer der Lagerung, bei mehrfachen Einfrier- und Auftauvorgängen und mechanischer Streßbeanspruchung hinsichtlich Gehalt, Zersetzungsprodukten und Aggregaten stabil. Sie kann bei Kühlschranktemperatur (2-8°C) über einen Zeitraum von mindestens 3 Monate bis zu einem Zeitraum von 4 Jahren stabil gelagert werden. Überraschenderweise ist die erfindungsgemäße Zubereitung auch bei höheren Temperaturen und Luftfeuchtigkeiten, beispielsweise bei einer Temperatur von 25°C und 60% relativer Luftfeuchtigkeit über einen Zeitraum bis 2 Jahre, beziehungsweise bei einer Temperatur von 40°C und 75% relativer Luftfeuchtigkeit über einen Zeitraum von 3 Monaten stabil lagerbar.

Als Aminosäure kann die Zubereitung basische Aminosäuren, wie z.B. Arginin, Histidin, Ornithin, Lysin, oder neutrale Aminosäuren, wie z.B. Glycin, Methionin Isoleucin, Leucin und Alanin, bzw. aromatische Aminosäuren, wie z.B. Phenylalanin, Tyrosin oder Tryptophan, enthalten. Basische Aminosäuren werden vorzugsweise in Form ihrer anorganischen Salze eingesetzt (vorteilhaft in Form der Salzsäuresalze, d.h. als Aminosäurehydrochloride). Als Aminosäure bevorzugt eingesetzt wird jeweils deren L-Form. Besonders bevorzugt enthält die erfindungsgemäße Zubereitung als Aminosäure L-Arginin, Glycin oder L-Methionin.

Die Zubereitung enthält die Aminosäure in einer Konzentration von 2 bis 200 mmol/l, bevorzugt 50 bis 150 mmol/l. Besonderes bevorzugt sind ca. 100 mmol/l Aminosäure enthalten.

Als Tenside einsetzbar sind alle in pharmazeutischen Zubereitungen üblicherweise verwendeten Tenside, vorzugsweise Polyethylen-Sorbitan-Fettsäureester und Polyoxyethylen-Polyoxypropylen-Copolymere. Polyethylen-Sorbitan-Fettsäureester sind auch unter dem Warenzeichen Tween bekannt. Geeignete Polyethylen-Sorbitan-Fettsäureester sind insbesondere Polyoxyethylen(20)-sorbitanmonolaurat, Polyoxyethylen(20)-sorbitanmonopalmitat und Polyoxyethylen(20)-sorbitanmonostearat. Bevorzugt sind Polyoxyethylen(20)-sorbitanmonolaurat und Polyoxyethylen(20)-sorbitanmonooleat, hiervon besonders bevorzugt ist Polyoxyethylen(20)-sorbitanmonooleat. Polyoxyethylen-Polyoxypropylen-Copolymere sind auch unter dem Warenzeichen Poloxamer bekannt. Als Polyoxyethylen-Polyoxypropylen-Copolymer besonders bevorzugt ist Poloxamer 407 (CAS 9003-11-6).

Die Tenside können in der Formulierung in einer Konzentration von 0,001 % bis 1,0 Gew.-% enthalten sein. Sind Polyoxyethylensorbitan-Fettsäureester als Tenside enthalten, sind diese bevorzugt in einer Menge von 0,005 bis 0,1 Gew.-% enthalten, besonders bevorzugt ist eine Menge von ca. 0,01 Gew.-%. Sind Polyoxyethylen-Polyoxypropylen-Copolymere enthalten, sind diese bevorzugt in einer Menge von 0,01 bis 0,5 Gew.-% enthalten, besonders bevorzugt sind ca. 0,1 Gew.-%.

Um die Verträglichkeit bei parenteraler Verabreichung zu erhöhen, liegt die Osmolalität vorzugsweise im isotonischen Bereich, d. h. bei einer Osmolalität von ca. 250 bis 350 mOsmol/kg. Die Zubereitung kann dann weitgehend schmerzfrei intravenös, intraarteriell und auch subkutan direkt verabreicht werden.

Nach einer vorteilhaften Ausführungsform enthält die erfindungsgemäße Zubereitung daher zusätzlich ein Isotonisierungsmittel, bevorzugt ein physiologisch verträgliches Salz, wie beispielsweise Natriumchlorid oder Kaliumchlorid, oder ein physiologisch verträgliches Polyol, wie beispielsweise Glukose oder Glycerin, in einer zur Isotonisierung erforderlichen Konzentration. Gegenstand der Erfindung ist daher weiterhin eine wässrige Zubereitung enthaltend einen anti-EGFR-Antikörper, einen Puffer, eine Aminosäure, ein Tensid sowie ein Isotonisierungsmittel in einer zur Isotonisierung erforderlichen Konzentration. Besonders bevorzugt ist Natriumchlorid als Isotonisierungsmittel enthalten.

Darüber hinaus können die erfindungsgemäßen Lösungen weitere physiologisch verträgliche Hilfsstoffe, wie z.B. Antioxidantien wie Ascorbinsäure oder Glutathion, Konservierungsmittel wie Phenol, m-Cresol, Methyl- oder Propylparaben, Chlorbutanol, Thiomersal oder Benzalkoniumchlorid, Polyethylenglykole (PEG) wie PEG 400, PEG 3000, 3350, 4000 oder 6000, Disaccharide wie Trehalose oder Saccharose, oder Cyclodextrine wie Hydroxypropyl-β-cyclodextrin, Sulfobutylethyl-βcyclodextrin, α-Cyclodextrin oder γ-Cyclodextrin, enthalten.

Nach einer besonders vorteilhaften Ausführungsform der Erfindung enthält die wässrige Zubereitung ca. 5 mg/ml Cetuximab oder EMD 72000, ca. 10 mmol/l Citrat- oder Histidinpuffer mit einem pH-Wert von ca. 5,5, ca. 100 mmol/l Glycin, Arginin oder L-Methionin, ca. 100 mmol/l Natriumchlorid sowie ca. 0,01 % Polyoxyethylen(20)-sorbitanmonooleat.

Die wässrige Zubereitung kann hergestellt werden, indem man einer den anti-EGFR-Antikörper enthaltenden Lösung die genannten Hilfsstoffe zugefügt werden. Zweckmäßigerweise wird hierzu einer Lösung mit einer definierten Konzentration an anti-EGFR-Antikörper, wie sie bei dessen Herstellung gewonnen wird, mit definierten Volumina an Stammlösungen, die die genannten weiteren Hilfsstoffe in definierter Konzentration enthalten, versetzt und gegebenenfalls mit Wasser oder Pufferlösung auf die vorberechnete Konzentration verdünnt. Alternativ können die Hilfsstoffe der den anti-EGFR-Antikörper enthaltenden Ausgangslösung auch als Feststoffe zugesetzt werden. Liegt der anti-EGFR-Antikörper als Feststoff, beispielsweise als Lyophilisat, vor, kann die erfindungsgemäße Zubereitung hergestellt werden, indem jeweilige Antikörper zunächst in Wasser oder einer einen oder mehrere der weiteren Hilfsstoffe enthaltenden wässrigen Lösung gelöst und anschließend mit den jeweils erforderlichen Mengen an die weiteren Hilfsstoffe enthaltenden Stammlösungen, mit den weiteren Hilfsstoffen in fester Form und/oder Wasser versetzt werden. Zweckmäßigerweise kann der anti-EGFR-Antikörper auch direkt in einer alle weiteren Hilfsstoffe enthaltenden Lösung gelöst werden.

Vorteilhaft kann einer oder mehrere der in der erfindungsgemäßen Zubereitung enthaltenen Hilfsstoffe bereits während oder zum Schluss des Herstellungsverfahrens des jeweiligen EGFR-Antikörpers zugegeben werden. Bevorzugt kann dies dadurch erfolgen, indem dem anti-EGFR-Antikörper im letzten Schritt der nach seiner Herstellung erfolgenden Aufreinigung direkt in einer einen, mehrere oder alle weiteren Hilfsstoffe enthaltenden wässrigen Lösung gelöst wird. Dann müssen zur Herstellung der Zubereitung die jeweiligen weiteren Inhaltsstoff/e nur noch in jeweils geringerer Menge und/oder gar nicht zugesetzt werden. Besonders bevorzugt ist, wenn der jeweilige Inhaltsstoff im letzten Schritt der nach seiner Herstellung erfolgenden Aufreinigung direkt in einer alle weiteren Hilfsstoffe enthaltenden wässrigen Lösung gelöst wird.
Soweit die den jeweiligen Antikörper sowie die Hilfsstoffe enthaltende Lösung noch nicht den gewünschten pH-Wert aufweist, wird dieser durch Zugabe einer Säure bzw. Base eingestellt, wobei vorzugsweise die bereits im Puffer-System enthaltene Säure bzw. Base Verwendung findet. Anschließend wird sterilfiltriert.

Die erfindungsgemäße wässrige Zubereitung kann vorteilhaft zur Behandlung von Tumorerkrankungen eingesetzt werden.

Die Beispiele, ohne darauf beschränkt zu sein, erläutern die Erfindung.

### Beispiel 1 (Vergleichsbeispiel 1)

Wässrige Lösung enthaltend:
2 mg/ml Cetuximab
10 mmol/l Natriumphosphatpuffer pH 7,2
145 mmol/l Natriumchlorid

Die Herstellung erfolgt durch Mischung definierter Volumina von die jeweiligen Hilfsstoffe in definierter Konzentration enthaltenden wässrigen Lösungen. Folgende Lösungen werden verwendet:
Lösung A (Wirkstofflösung) enthaltend:
18 mg/ml Cetuximab
10 mmol/l Natriumphosphatpuffer pH 7,2
(bestehend aus 2,07 g/l Dinatriumhydrogenphosphat-7-hydrat und 0,31 g/l Natriumdihydrogenphosphat-Monohydrat)
145 mmol/l Natriumchlorid

(Die Lösung wird erhalten, indem der Wirkstoff im letzten Schritt der nach dessen Herstellung erfolgenden Wirkstoffaufreinigung gegen Lösung B mit Hilfe der Tangentialflußfiltration umgepuffert wird.)
Lösung B (Puffer/Salz-Lösung)
entspricht Lösung A, enthält jedoch keinen Wirkstoff

Zur Herstellung der Vergleichslösung 1 werden 1,11 Volumenteile Lösung A und 8,89 Volumenteile Lösung B miteinander vereinigt.

Die zubereitete Lösung wird vor der Abfüllung mit einem Sterilfilter filtriert und in Injektionsgläser abgefüllt. Anschließend werden die Injektionsgläser mit Stopfen verschlossen und gebördelt.

### Beispiel 2 (Vergleichsbeispiel 2)

Wässrige Lösung enthaltend:
2 mg/ml Cetuximab
10 mmol/l Natriumphosphatpuffer pH 7,2
145 mmol/l Natriumchlorid
0,01 Gew.-% Polyoxyethylen(20)-sorbitanmonooleat

Die Herstellung erfolgt durch Mischung definierter Volumina von die jeweiligen Inhaltsstoffe in definierter Konzentration enthaltenden wässrigen Lösungen. Neben Lösung A wird folgende Lösung verwendet.

Lösung C (Puffer/Salz-Lösung enthaltend Polyoxyethylen(20)sorbitan-monooleat)
entspricht Lösung B, enthält jedoch zusätzlich
0,0125 Gew.-% Polyoxyethylen(20)sorbitanmonooleat.

Zur Herstellung der Vergleichslösung 2 werden 1,11 Volumenteile Lösung A mit 8,89 Volumenteile Lösung C miteinander vereinigt.

Die zubereitete Lösung wird vor der Abfüllung mit einem Sterilfilter filtriert und in Injektionsgläser abgefüllt. Anschließend werden die Injektionsgläser mit Stopfen verschlossen und gebördelt.

### Beispiel 3 (erfindungsgemäße Formulierung)

5 mg/ml Cetuximab
10 mmol/l Citratpuffer pH 5,5
100 mmol/l Glycin
100 mmol/l Natriumchlorid
0,01 Gew.-% Polyoxyethylen(20)sorbitanmonooleat

Die Herstellung erfolgt durch Mischung definierter Volumina von die jeweiligen Inhaltsstoffe in definierter Konzentration enthaltenden wässrigen Lösungen.

Lösung D (Wirkstofflösung in einem Citrat-Puffer)
16 mg/ml Cetuximab
10 mol/l Citratpuffer pH 5,5
(bestehend aus 2,1014 g/l Citronensäure-Monohydrat)
(Die Lösung wird erhalten, indem der Wirkstoff im letzten Schritt der nach dessen Herstellung erfolgenden Wirkstoffaufreinigung gegen Lösung E mit Hilfe der Tangentialflußfiltration umgepuffert wird.)

Lösung E (Puffer-Lösung):
entspricht Lösung D, enthält jedoch keinen Wirkstoff.

Lösung F (Puffer/Salz-Lösung):
entspricht Lösung E, enthält jedoch
145,5 mmol/l Glycin,
145,5 mmol/l Natriumchlorid und
0,015 Gew.-% Polyoxyethylen(20)sorbitanmonooleat.

Zur Herstellung der erfindungsgemäßen Formulierung werden 3,125 Volumenteile Lösung D und 6,875 Volumenteile Lösung F miteinander vereinigt.

Die zubereitete Lösung wird vor der Abfüllung mit einem Sterilfilter filtriert und in Injektionsgläser abgefüllt. Anschließend werden die Injektionsgläser mit Stopfen verschlossen und gebördelt.

### Beispiel 4

Analog dem Verfahren der vorbeschriebene Beispiele werden folgende Lösungen hergestellt:
Beispiel 4.1, Lösung enthaltend:
   5 mg/ml Cetuximab
   100 mmol/l Glycin
   0,01 Gew.-% Polyoxyethylen(20)-sorbitanmonooleat
   10 mmol/l Citratpuffer pH 5,5
   (bestehend aus 2,9410 g/l tri-Natriumcitrat-Dihydrat)
Beispiel 4.2, Lösung enthaltend:
   5 mg/ml Cetuximab
   100 mmol/l Glycin
   100 mmol/l Natriumchlorid
   0,01 Gew.-% Polyoxyethylen(20)-sorbitanmonooleat
   10 mmol/l Citratpuffer pH 5,5
   (bestehend aus 2,1014 g/l Citronensäure-Monohydrat)
Beispiel 4.3, Lösung enthaltend:
   5 mg/ml EMD 72000
   100 mmol/l Glycin
   100 mmol/l Natriumchlorid
   0,01 Gew.-% Polyoxyethylen(20)-sorbitanmonooleat
   10 mmol/l Citratpuffer pH 5,5
   (bestehend aus 2, 1014 g/l Citronensäure-Monohydrat)
Beispiel 4.4, Lösung enthaltend:
   5 mg/ml Cetuximab
   100 mmol/l L-Methionin
   0,01 Gew.-% Polyoxyethylen(20)-sorbitanmonooleat
   10 mmol/l Citratpuffer pH 5,5
   (bestehend aus 2,1014 g/l Citronensäure-Monohydrat)
Beispiel 4.5, Lösung enthaltend:
   5 mg/ml Cetuximab
   100 mmol/l Glycin
   0,01 Gew.-% Polyoxyethylen(20)-sorbitanmonooleat
   10 mmol/l Acetatpuffer pH 5,5
   (bestehend aus 1,3608 g/l Natriumacetat-Trihydrat
Beispiel 4.6, Lösung enthaltend:
   5 mg/ml Cetuximab
   100 mmol/l Glycin
   0,01 Gew.-% Polyoxyethylen(20)-sorbitanmonooleat
   10 mmol/l Histidinpuffer pH 5,5
   (bestehend aus 2,069 g/l L-Histidinmonohydrochlorid-Monohydrat)
Beispiel 4.7, Lösung enthaltend:
   5 mg/ml Cetuximab
   100 mmol/l Glycin
   0,01 Gew.-% Polyoxyethylen(20)-sorbitanmonolaurat
   10 mmol/l Citratpuffer pH 5,5
   (bestehend aus 2,1014 g/l Citronensäure-Monohydrat
Beispiel 4.8, Lösung enthaltend:
   5 mg/ml Cetuximab
   100 mmol/l Glycin
   0,1 Gew.-% Polyoxyethylen-Polyoxypropylen-Copolymer 407 (Poloxamer 407)
   10 mmol/l Citratpuffer pH 5,5
   (bestehend aus 2,1014 g/l Citronensäure-Monohydrat)

### Beispiel 5

Die Stabilität der erfindungsgemäßen Formulierung wurde in einem Stresstest geprüft. Hierzu wurden Vials enthaltend die Lösung gemäß Beispiel 3 sowie zu Vergleichszwecken Vials enthaltend Lösung gemäß Beispiel 1 und 2 bei 25°C und 60% relativer Luftfeuchte und 40°C und 75% relativer Luftfeuchte eingelagert. Zusätzlich wurden Vials der Beispiele 1, 2 und 3 fünf Tage auf einer Schüttelapparatur mit einer Schüttelfrequenz von 150 min⁻¹ bei Raumtemperatur geschüttelt, sowie dreimal in Folge bei -20°C eingefroren und anschließend bei +5°C wieder aufgetaut. Vor Einlagerung sowie nach definierten Lagerzeiten wurden die Vials visuell bei direkter Anstrahlung mit einer Kaltlichtquelle beurteilt und die Absorption der Lösungen bei 350 nm bestimmt, die ein Maß für die Trübung darstellt. Um den Einfluss der Lagerung bzw. Behandlung zu verdeutlichen, wurde jeweils die relative Trübung bezogen auf den Ausgangswert berechnet. Weiterhin wurden die Vials hinsichtlich des Gehaltes an Cetuximab, Aggregaten und Zersetzungsprodukten mittels HPLC-Gelfiltration untersucht.

Die Ergebnisse der Stabilitätsuntersuchungen sind in Tabelle 1 dargestellt.

**Tabelle 1: Zusammenfassung der Stabilitätsdaten der erfindungsgemäßen Formulierung (Beispiel 3) und der beiden Vergleichslösungen (Beispiele 1 und 2)**

| Prüfungs-lösung | Lagerung [Zeit / Bedingungen] | Cetuxi- Aggremab [%] | gate [%] | Zersetzungs-produkte [%] | Trübung bei λ= 350 nm | relative Trübung bei λ= 350 nm | visuelle Beurteilung |
|---|---|---|---|---|---|---|---|
| Beispiel 1 | 0 Wochen | 99,67 | 0,12 | 0,22 | 0,005 | 1,00 | kleine Partikel, geringe Anzahl, Klar |
| Beispiel 1 | 8 Wochen 25°C/60% r.F. | 98,99 | 0,28 | 0,73 | 0,0081 | 1,62 | große Partikel, große Anzahl, Klar |
| Beispiel 1 | 8 Wochen 40°C/75% r.F. | 95,08 | 3,23 | 1,69 | 0,0235 | 4,70 | große Partikel, große Anzahl, Klar |
| Beispiel 1 | 5 Tage Schütteln bei 150 Upm und RT | 99,60 | 0,17 | 0,24 | 0,829 | 165,80 | sehr große Partikel, sehr hohe Partikelanzahl, Trübung |
| Beispiel 1 | 3 Frier-Tau-Zyklen zwischen -20°C und +5°C | 99,68 | 0,14 | 0,18 | 0,0089 | 1,78 | große Partikel, hoher Partikel-gehalt, leichte Trübung |
| | | | | | | | |
| Beispiel 2 | 0 Wochen | 99,62 | 0,18 | 0,21 | 0,0048 | 1,00 | keine Partikel, Klar |
| Beispiel 2 | 8 Wochen 25°C/60% r.F. | 99,02 | 0,28 | 0,70 | 0,0071 | 1,48 | kleine Partikel, geringe Anzahl, Klar |
| Beispiel 2 | 8 40°C/75% r.F. | 93,95 | 4,34 | 1,72 | 0,0241 | 5,02 | kleine Partikel, geringe Anzahl, Klar |
| Beispiel 2 | 5 Tage Schütteln bei 150 Upm und RT | 99,51 | 0,26 | 0,23 | 0,0075 | 1,56 | keine Partikel, Klar |
| Beispiel 2 | 3 Frier-Tau-Zyklen zwischen -20°C und +5°C | 99,61 | 0,21 | 0,18 | 0,0064 | 1,48 | keine Partikel, klar |
| | | | | | | | |
| Beispiel 3 | 0 Wochen | 99,72 | 0,15 | 0,14 | 0,018 | 1,00 | keine Partikel, Klar |
| Beispiel 3 | 8 Wochen 25°C/60% r.F. | 99,38 | 0,18 | 0,44 | 0,020 | 1,08 | kleine Partikel, geringe Anzahl, Klar |
| Beispiel 3 | 8 Wochen 40°C/75% r.F. | 98,15 | 0,46 | 1,40 | 0,030 | 1,63 | kleine Partikel, geringe Anzahl, Klar |
| Beispiel 3 | 5 Tage Schütteln bei 150 Upm und RT | 99,15 | 0,70 | 0,15 | 0,019 | 1,04 | keine Partikel, Klar |
| Beispiel 3 | 3 Frier-Tau-Zyklen zwischen -20°C und +5°C | 99,75 | 0,14 | 0,12 | 0,018 | 1,00 | keine Partikel, klar |

Die Ergebnisse zeigen deutlich, dass die erfindungsgemäße Formulierung gegenüber den Vergleichslösungen des Standes der Technik eine deutlich erhöhte Stabilität aufweist.

## Patentansprüche

1. Wässrige Zubereitung bestehend aus einem Lösungsmittel, das zumindest zum Teil aus Wasser besteht, einem anti-EGFR (epidermal growth factor receptor)-Antikörper, einem Puffer, einer Aminosäure, einem Tensid und Natriumchlorid als Isotonisierungsmittel

2. Zubereitung gemäß Anspruch 1, **dadurch gekennzeichnet, dass** er Antikörper Cetuximab oder EMD 72000 bzw. eines der diesen entsprechenden murinen, humanisierten oder chimären Antikörperanaloga ist

3. Zubereitung gemäß Anspruch 2, **dadurch gekennzeichnet, dass** der Antikörper Cetuximab oder EMD 72000 ist

4. Zubereitung gemäß einem oder mehreren der Ansprüche 1 bis 3, **dadurch gekennzeichnet, dass** der Puffer aus einem oder mehreren Citrat-Saiz/en, Acetat-Salz/en, Histidin-Salz/en, Succinat-Salz/en, Malat-Salz/en, Phosphat-Salz/en oder Lactat-Salz/en und/oder deren jeweilige/n freie/n Säure/n bzw. Base/n oder aus einer Mischung aus einem oder mehreren der verschiedenen Salze und/oder deren Säure/n bzw. Base/n besteht

5. Zubereitung gemäß Anspruch 4, **dadurch gekennzeichnet, dass** der Puffer aus einem oder mehreren Citrat-Satz/en und/oder dessen freie Säure, AcetatSalz/en und/oder dessen freie Säure oder aus L-Histidin und/oder einem Säureadditionssalz hiervon besteht

6. Zubereitung gemäß einem oder mehreren der Ansprüche 1 bis 5, **dadurch gekennzeichnet, dass** die Aminosäure L-Arginin, Glycin oder L-Methionin ist

7. Zubereitung gemäß einem oder mehreren der Ansprüche 1 bis 6, **dadurch gekennzeichnet, dass** das Tensid ein Polyethylen-Sorbitan-Fettsäureester oder ein Polyoxyethylen-Polyoxypropylen-Copolymer ist

8. Zubereitung gemäß Anspruch 7, **dadurch gekennzeichnet**, **dadurch gekennzeichnet, dass** das Tensid Polyoxyethylensorbitan-Fettsäureester Polyoxyethylen(20)-sorbitanmonooleat oder Polyoxyethylen(20)-sorbitanmonolaurat ist

9. Zubereitung gemäß Anspruch 7, **dadurch gekennzeichnet, dass** das Tensid Poloxamer 407 ist

10. Zubereitung gemäß einem oder mehreren der Ansprüche 1 bis 9, **dadurch gekennzeichnet, dass** als Lösungsmittel Wasser oder eine Mischung aus Wasser mit Alkoholen enthalten ist

11. Zubereitung gemäß Anspruch 10, **dadurch gekennzeichnet, dass** als Alkohol Ethanol, Propanol, Propandiol und/oder Glycerol enthalten ist

12. Zubereitung gemäß einem oder mehreren der Ansprüche 1 bis 11, **dadurch gekennzeichnet, dass** diese einen pH-Wert von 5 - 7, vorzugsweise von pH 5,2 bis pH 6,0, aufweist

13. Zubereitung nach Anspruch 12, **dadurch gekennzeichnet, dass** diese einen pH-Wert von cirka 5,5 aufweist

14. Wässrige Zubereitung enthaltend ca. 5 mg/ml Cetuximab oder EMD 72000, ca. 10 mmol/l Citrat- oder Histidinpuffer, ca. 100 mmol/l Glycin, L-Arginin oder L-Methionin, ca. 100 mmol/l Natriumchlorid sowie ca. 0,01 % Polyoxyethylen(20)-sorbitanmonooleat mit einem pH-Wert von ca. 5,5

15. Verfahren zur Herstellung einer pharmazeutischen Zubereitung gemäß einem oder mehreren der Ansprüche 1 bis 14, **dadurch gekennzeichnet, dass** man eine wässrige Zubereitung enthaltend den anti-EGFR-Antikörper einer die genannten Hilfsstoffe zufügt

16. Verwendung der Zubereitung gemäß einem oder mehreren der Ansprüche 1 bis 14 zur Herstellung eines Arzneimittels zur Behandlung von Tumorerkrankungen

## Claims

1. Aqueous composition consisting of a solvent which consists at least partly of water, an anti-EGFR (epidermal growth factor receptor) antibody, a buffer, an amino acid, a surfactant and sodium chloride as isotonic agent.

2. Composition according to Claim 1, **characterised in that** the antibody is cetuximab or EMD 72000 or one of the murine, humanised or chimeric antibody analogues corresponding thereto.

3. Composition according to Claim 2, **characterised in that** the antibody is cetuximab or EMD 72000.

4. Composition according to one or more of Claims 1 to 3, **characterised in that** the buffer consists of one or more citrate salt(s), acetate salt(s), histidine salt(s), succinate salt(s), malate salt(s), phosphate salt(s) or lactate salt(s) and/or the respective free acid(s) or base(s) thereof or a mixture of one or more of the various salts and/or the acid(s) or base(s) thereof.

5. Composition according to Claim 4, **characterised in that** the buffer consists of one or more citrate salt(s) and/or the free acid thereof, acetate salt(s) and/or the free acid thereof or L-histidine and/or an acid-addition salt thereof.

6. Composition according to one or more of Claims 1 to 5, **characterised in that** the amino acid is L-arginine, glycine or L-methionine.

7. Composition according to one or more of Claims 1 to 6, **characterised in that** the surfactant is a polyethylene sorbitan fatty acid ester or a polyoxyethylene-polyoxypropylene copolymer.

8. Composition according to Claim 7, **characterised in that** the polyoxyethylene sorbitan fatty acid ester surfactant is polyoxyethylene (20) sorbitan monooleate or polyoxyethylene (20) sorbitan monolaurate.

9. Composition according to Claim 7, **characterised in that** the surfactant is Poloxamer 407.

10. Composition according to one or more of Claims 1 to 9, **characterised in that** the solvent present is water or a mixture of water with alcohols.

11. Composition according to Claim 10, **characterised in that** the alcohol present is ethanol, propanol, propanediol and/or glycerol.

12. Composition according to one or more of Claims 1 to 11, **characterised in that** it has a pH of 5 - 7, preferably from pH 5.2 to pH 6.0.

13. Composition according to Claim 12, **characterised in that** it has a pH of about 5.5.

14. Aqueous composition comprising about 5 mg/ml of cetuximab or EMD 72000, about 10 mmol/l of citrate or histidine buffer, about 100 mmol/l of glycine, L-arginine or L-methionine, about 100 mmol/l of sodium chloride and about 0.01% of polyoxyethylene (20) sorbitan monooleate and having a pH of about 5.5.

15. Process for the preparation of a pharmaceutical composition according to one or more of Claims 1 to 14, **characterised in that** an aqueous composition comprising the anti-EGFR antibody is added to one of the said auxiliaries.

16. Use of the composition according to one or more of Claims 1 to 14 for the preparation of a medicament for the treatment of tumour diseases.

## Revendications

1. Composition aqueuse constituée d'un solvant qui est constitué au moins partiellement d'eau, d'un anticorps anti-EGFR (récepteur du facteur de croissance de l'épiderme), d'un tampon, d'un acide aminé, d'un agent tensioactif et de chlorure de sodium comme agent isotonique.

2. Composition selon la revendication 1, **caractérisée en ce que** l'anticorps est le cetuximab ou l'EMD 72000 ou l'un des analogues d'anticorps murins, humanisés ou chimères leur correspondant.

3. Composition selon la revendication 2, **caractérisée en ce que** l'anticorps est le cetuximab ou l'EMD 72000.

4. Composition selon l'une ou plusieurs des revendications 1 à 3, **caractérisée en ce que** le tampon est constitué d'un ou plusieurs sel(s) de citrate, sel(s) d'acétate, sel(s) d'histidine, sel(s) de succinate, sel(s) de malate, sel(s) de phosphate ou sel(s) de lactate et/ou de leur(s) acide(s) ou base(s) libre(s) respectif(s) ou d'un mélange d'un ou plusieurs des divers sels et/ou de leur(s) acide(s) ou base(s).

5. Composition selon la revendication 4, **caractérisée en ce que** le tampon est constitué d'un ou plusieurs sel(s) de citrate et/ou de leur acide libre, sel(s) d'acétate et/ou de leur acide libre ou de L-histidine et/ou de l'un de ses sels d'addition d'acide.

6. Composition selon l'une ou plusieurs des revendications 1 à 5, **caractérisée en ce que** l'acide aminé est la L-arginine, la glycine ou la L-méthionine.

7. Composition selon l'une ou plusieurs des revendications 1 à 6, **caractérisée en ce que** l'agent tensioactif est un ester d'acide gras et de sorbitan polyéthyléné ou un copolymère de polyoxyéthylène-polyoxypropylène.

8. Composition selon la revendication 7, **caractérisée en ce que** l'agent tensioactif d'ester d'acide gras et de sorbitan polyéthyléné est le monooléate de polyoxyéthylène (20) sorbitan ou le monolaurate de polyoxyéthylène (20) sorbitan.

9. Composition selon la revendication 7, **caractérisée en ce que** l'agent tensioactif est le Poloxamer 407.

10. Composition selon l'une ou plusieurs des revendications 1 à 9, **caractérisée en ce que** le solvant présent est l'eau ou un mélange d'eau et d'alcools.

11. Composition selon la revendication 10, **caractérisée en ce que** l'alcool présent est l'éthanol, le propanol, le propanediol et/ou le glycérol.

12. Composition selon l'une ou plusieurs des revendications 1 à 11, **caractérisée en ce qu'**elle possède un pH de 5 - 7, préférablement un pH allant de 5,2 à 6,0.

13. Composition selon la revendication 12, **caractérisée en ce qu'**elle possède un pH d'environ 5,5.

14. Composition aqueuse comprenant environ 5 mg/ml de cetuximab ou d'EMD 72000, environ 10 mmol/l de tampon citrate ou histidine, environ 100 mmol/l de glycine, de L-arginine ou de L-méthionine, environ 100 mmol/l de chlorure de sodium et environ 0,01 % de monooléate de polyoxyéthylène (20) sorbitan et possédant un pH d'environ 5,5.

15. Procédé de préparation d'une composition pharmaceutique selon l'une ou plusieurs des revendications 1 à 14, **caractérisé en ce qu'**une composition aqueuse comprenant l'anticorps anti-EGFR est ajoutée à l'un desdits auxiliaires.

16. Utilisation de la composition selon l'une ou plusieurs des revendications 1 à 14 pour la préparation d'un médicament destiné au traitement de maladies tumorales.
